# EUROPEAN PATENT APPLICATION

(11) **EP 2 292 572 A1**
(43) Date of publication of application: **09.03.2011**
(21) Application number: 09169647.6
(22) Date of filing: 07.09.2009
(51) Int. Cl.: C07B 63/04, C07C 7/10, C07C 7/11, C10G 21/20

(54) **Process for the separation of aromatic compounds from a mixture**

(71) Applicant: Shell Internationale Research Maatschappij B.V., 2596 HR Den Haag (NL)
(72) Inventor: The designation of the inventor has not yet been filed
(74) Representative: Matthezing, Robert Maarten

(57) **Abstract**

Aromatic compounds are separated from a mixture comprising such aromatic compounds and aliphatic hydrocarbons in a process which comprises;
(a) contacting the mixture with an ionic liquid in an extraction zone to yield an aromatics-rich ionic liquid stream and an aromatics-depleted aliphatic stream;
(b) feeding at least a portion of the aromatics-rich ionic liquid stream to a stripping zone;
(c) stripping the aromatics-rich ionic liquid with a non-aqueous stripping gas to obtain an aromatics-rich hydrocarbon stream and a purified ionic liquid stream; and
(d) recycling the purified ionic liquid stream to the extraction zone.

## Description

The present invention relates to a process for the separation of aromatic compounds from a mixture comprising such aromatic compounds and aliphatic hydrocarbons.

In refinery processes a number of streams become available that contain aromatic and aliphatic compounds. It is often desirable to separate the aromatic compounds from the aliphatic compounds. Separation of aromatic compounds may be desirable when the aromatic compounds contain heteroatoms such as sulphur or nitrogen. Although removal of such heteroatom-containing compounds is commonly achieved by hydrodesulphurisation and hydrodenitrification processes, it is also possible to remove such compounds by extraction with ionic liquids.

Such a process has been described in EP-A 1854786. This known process relates to the extraction of aromatic compounds, such as dibenzothiophene and derivatives thereof, from a mixture with at least one aliphatic hydrocarbon by contacting the mixture with an ionic liquid, which is a salt in the liquid state having a cation comprising an aromatic nitrogen-containing heterocyclic ring system. The ring system comprises an electron-withdrawing substituent, such as a cyano group. The nature of the anion is stated not to be crucial.

In WO-A 01/40150 the extraction of aromatics is disclosed in which extraction an ionic liquid is used, which ionic liquid must be non-neutral. Neutral has been defined on the basis of Lewis acid and Lewis base. At equimolar proportion in case of a nitrogen-containing cation and aluminium trichloride as function for the anion the resulting composition is neither acidic nor basic. Hence, an ionic liquid is neutral in the sense of WO-A 01/40150 if the anion and cation are equimolar and no acidic or basic character is present.

Various refinery streams may comprise mixtures of aliphatic and aromatic compounds. Examples include naphtha streams that have been subjected to reforming, e.g., with a platinum catalyst. It may be desirable to obtain pure aromatic compounds from such streams. US-B 7019188 relates to a process for the extraction of olefins and aromatics using an ionic liquid that comprises a heterocyclic cationic group, e.g., 1-butyl-3-methylimidazolium, and tetrafluoroborate as anion.

The above documents describe small-scale experiments and do not address the design of a commercial-scale process. A proposal for a commercial design has been provided by S T Anjan, Chemical Engineering Progress, 2006, 102 (12) 30-39. This article describes a conventional aromatic extraction process comprising a liquid-liquid extraction column, a water wash for the aliphatic phase and a steam stripper for the aromatics-solvent mixture obtained. The solvent is subsequently recovered in a vacuum flash column. The article already indicates that this design has several drawbacks. Therefore, in the article another design is proposed for processes that use ionic liquids. This new design retains the liquid-liquid extraction column and the water wash for the aliphatic hydrocarbon fraction. The mixture of ionic liquid and aromatics is separated in a flash vessel to obtain the aromatics as top product and the ionic liquid as the bottom product that is subsequently recycled to the extraction column. This new design requires that all aromatic compounds need to be evaporated which requires much energy. The new design also ignores an additional drawback, viz., that certain types of ionic liquids decompose on contact with water or steam, e.g. ionic liquids containing chloroaluminate, tetrafluoroborate or hexafluorophosphate anions. Since this new design retains the water wash, this additional drawback is not overcome.

The present invention aims at overcoming the above-mentioned drawbacks.

Accordingly, the present invention provides a process for the separation of aromatic compounds from a mixture comprising such aromatic compounds and aliphatic hydrocarbons, which process comprises;
(a) contacting the mixture with an ionic liquid in an extraction zone to yield an aromatics-rich ionic liquid stream and an aromatics-depleted aliphatic stream;
(b) feeding at least a portion of the aromatics-rich ionic liquid stream to a stripping zone;
(c) stripping the aromatics-rich ionic liquid with a non-aqueous stripping gas to obtain an aromatics-rich hydrocarbon stream and a purified ionic liquid stream; and
(d) recycling the purified ionic liquid stream to the extraction zone.

The aromatic compound that is separated from the mixture may be any aromatic organic compound. Examples include sulphur- and/or nitrogen-containing aromatic compounds that are found as contaminants in refinery streams. Such compounds can be benzothiophene compounds, more in particular alkyl-substituted dibenzothiophene compounds. Such compounds are sometimes referred to as refractory sulphides and are difficult to remove with mild hydrodesulphurisation. Therefore, such heteroatom-containing compounds may suitably be subjected to the process of the present invention in order to remove these contaminants from refinery streams. However, since the process is found to be excellently suitable to recover aromatic compounds in large proportions for use in, e.g., gasoline fuel or chemical conversions, it is, preferred that the aromatic compound to be separated is a hydrocarbon that does not comprise a heteroatom. Suitable compounds include hydrocarbon aromatics with 6 to 12 carbon atoms in the ring. Suitably, the aromatic compounds may comprise one or more C₁-C₆ substituents. Although the substituents may be unsaturated, it is preferred that the C₁-C₆ substituents are alkyl groups. The present process is excellently suitable for the removal of benzene, toluene, xylene (o-, m-, p- and mixtures) and naphthalene from hydrocarbon mixtures.

The process of the present invention may be appli-ed to a variety of feedstocks. Hence, the amounts of aliphatic and aromatic compounds in the mixture may vary within wide ranges. The process of the present invention is suitably applied to mixtures that comprise from 1 to 95 %wt of aromatic compounds, preferably 10 to 75%wt of aromatic compounds, based on the total weight of the mixture.

Ionic liquids are known in the art and are organic molten salts that are liquid at relatively low temperatures, suitably up to 150 °C. Suitable melting points may range from -100 to 100 °C, preferably from 0 to 80°C, most preferably below from 0 to 20 °C. Ionic liquids are generally known and described in, e.g., EP-A 1854786, WO-A 01/40150, US-B 7019188, US-A 4359596 and the references cited in US-B 7019188. The cationic moiety of the ionic liquid advantageously comprises a quaternary nitrogen or phosphorus atom. Hence, ionic liquids comprise ammonium and phosphonium compounds. Such compounds suitably comprise one or more organic substituent groups, in particular alkyl groups with 1 to 12 carbon atoms. Preferably, the phosphonium and ammonium moieties comprise three or four alkyl groups. Examples of suitable ammonium and phosphonium groups include trimethylammonium, tetramethyl ammonium, triethylammonium, tetraethylammonium, triethylphosphonium and tetraethylphosphonium. According to WO-A 01/40150 such moieties provide suitable ionic liquids. Since nitrogen-containing ionic liquids provide the better results, the ionic liquid preferably comprises a nitrogen-containing cationic moiety. In accordance with the teachings in US-B 7019188 and EP-A 1854786 the nitrogen-containing cationic moiety is preferably a heterocyclic group, more preferably, a heteroaromatic group. The ionic liquid can be formed by reacting the nitrogen-containing heterocyclic, or heteroaromatic, compound with an alkylating agent to form a quaternary ammonium salt and by reacting the salt with a Lewis acid to form the ionic liquid. Examples of nitrogen-containing heterocyclic rings that may be used in the formation of ionic liquids include pyrrole, pyrrolidine, imidazole, oxazole, piperidine, azepine, pyridine, pyrazine, pyrimidine, pyridazine, but also polycyclic rings, such as quinoline, isoquinoline, pteridine, indole, purine and carbazole. Since heteroaromatic compounds are preferred the nitrogen-containing ring is preferably selected from pyridine, imidazole, quinoline, isoquinoline, pteridine, pyrazine or purine.

When the cationic moiety comprises a nitrogen-containing heterocyclic group, this group is preferably substituted. Substituents are suitably positioned on the nitrogen atom, but advantageously, one or more substituents are also located on one or more of the carbon atoms in the ring of the heterocyclic group. The substituents can be selected from a wide group of organic and inorganic groups, including halides, cyano, nitro, trifluoromethyl, sulphide and alkylsulphide groups. Preferably, the substituents are hydrocarbyl groups, optionally interrupted by a one or more of oxygen, nitrogen and sulphur atoms. Such substituents have suitably from 1 to 16 carbon atoms. If the substituent has too many carbon atoms the melting point of the salt may increase so that it may require too high a temperature to become economically useful in the process of the present invention. Therefore, the substituent on the cationic moiety advantageously has from 1 to 6 carbon atoms. The substituent need not necessarily be saturated. Especially, when a substituent is bound to a nitrogen atom in the ring, the substituent may be unsaturated, e.g., an allyl, butenyl, isobutenyl or pentenyl group. However, advantageously the substituents are saturated, more preferably, the substituent is an alkyl group with 1 to 6 carbon atoms. Therefore, the cationic moiety is preferably selected from the group consisting of (N-R₁)-pyridinium and 1-R₂₋3-R₃-imidazolium, wherein R₁, R₂ and R₃ are alkyl groups with 1 to 6 carbon atoms. When the alkyl substituent contains more than 3 carbon atoms, the alkyl group is suitably linear. Such pyridinium and imidazolium moieties may bear one or more further substituents. Suitably the pyridinium moiety may contain one or more further hydrocarbyl, preferably alkyl substituents with 1 to 6 carbon atoms. If the pyridinium moiety contains more than one substituent the melting point of the resulting salt tends to increase to undesirable levels. Therefore, the pyridinium group preferably contains up to one further substituent. The further substituent may be positioned on the o-, m-, or p-position, wherein the o- and m-positions are preferred, since such compounds tend to have the lower melting points. Imidazolium moieties may also comprise one or more hydrocarbyl, suitably alkyl substituents. The imidazolium moiety preferably contains not more than one further substituent. Preferably, the substituent is positioned on the 2-position since imidazolium compounds with an alkyl group on the 2-position tend to be more stable.

The anionic moiety of the ionic liquid can be selected from a wide range of ions. Anions may be selected from organic and inorganic ions. In general, larger, less symmetrical anions are more likely to result in an ionic liquid than smaller, symmetrical anions. Preferred anions for use in the present invention include alkyl or haloalkyl methanamides (for example tristrifluoromethanesulphonylamide), alkyl or haloalkyl sulphonylamides (for example bistrifluoromethane-sulphonylamide, also referred to as bis(trifyl)imide or bistriflamide), alkyl or haloalkyl sulphates (for example methylsulphate and octylsulphate), alkyl or haloalkyl sulphonates (for example methanesulphonate, trifluoromethanesulphonate and dioctylsulphosuccinate), carboxylates (for example methanoate, ethanoate, benzoate and lactate), dicyanamide ([N(CN)₂]-), azolates (for example 1,2,4-triazolate), phosphates or phosphonates (for example dimethylphosphate and hexafluorophosphate), perhalides, pseudohalides (for example cyanate and thiocyanide), and various fluorinated anions (for example tetrafluoroborate, perfluoroalkylfluorophosphates, and fluorinated borates). Preferably, the anionic moiety comprises an element selected from the Groups IB, IIA, IIB, IIIA and VIII of the Periodic Table of the Elements. More preferably, suitable elements are selected from the group consisting of boron, aluminium, gallium, indium, copper, zinc, cobalt, nickel and iron. The anionic moiety may contain as halogen fluorine, chlorine, bromine or iodine. Since the application of bromine and iodine may result in high melting temperatures and since fluorine may be environmentally undesirable the anionic moiety preferably comprises chlorine. Most preferably, the anionic moiety comprises iron and halogen, suitably chlorine. Excellent results are obtainable with ionic liquids having tetrachloroferrate as anion.

The contact between the mixture and the ionic liquid in the extraction zone can be carried out in any way known in the art. The two streams may be contacted with each other in a co-current way. Since the transfer of aromatic compounds from the mixture into the ionic liquid occurs better in a counter-current way, the streams are preferably contacted in a counter-current way. It is possible to incur a gas-liquid contact wherein the mixture has been rendered gaseous and subsequently put into contact with the ionic liquid. This operation is generally known as extractive distillation. Evidently, that means that the extraction zone has to be operated at elevated temperature. Although this operation is a feasible option, such an operation requires a significant energy input. The extraction zone is preferably operated in a liquid-liquid extraction mode. The extraction zone is, therefore, advantageously operated at a temperature of 20 to 100 °C, and a pressure of 0.1 to 20 bar. The skilled person can vary the period during which the contact takes place in accordance with the amount of aromatic compounds in the mixture, the nature of the ionic liquid and the desired purity. Preferably the contact in the extraction zone will be from 1 s to 10 hr, preferably from 30 s to 1 hr, depending on the type of contact equipment. A high-gravity rotating bed contactor, e.g., as the one described in US-B 7344126 or in D Saha, Heat and Mass Transfer, 45 (No. 4), 2009, pages 451-457, can be operated at very short contact times. A rotating disk contactor may take longer residence times, up to 1 hr or more.

The extraction operation yields an aromatics-depleted aliphatic stream. Generally, the aromatics-depleted aliphatic stream may comprise still some aromatic compounds and traces of ionic liquid. It may be advantageous to purify this stream. It may be feasible to purify the stream by fractionation or flashing. This operation is very suitable to separate the relatively low-boiling hydrocarbons from the relatively high boiling ionic liquids. Since the boiling points of at least some of the aliphatic hydrocarbons and aromatic compounds can be close the fractionation of these compounds may be difficult. It is, therefore, preferred to contact the aromatics-depleted aliphatic stream with an adsorbent to yield a purified aliphatic stream and fat adsorbent comprising ionic liquid and, optionally some aromatic compounds. The purified aliphatic stream can be recovered as product or be subjected to further treatment such as fractionation or purification, as desired.

The fat adsorbent is preferably freed from ionic liquid by contacting the fat adsorbent with a liquid stream capable of dissolving the ionic liquid. The liquid stream advantageously comprises an aromatic hydrocarbon, since this dissolves the ionic liquid easily. It is most preferred to apply aromatic compounds that are the same as those, which are being separated in the present process. The aromatic compound may be a pure chemical compound, but it may also consist of a mixture of aromatic hydrocarbons, e.g., a mixture of benzene, toluene and/or one or more xylenes. The liquid stream is suitably used at ambient temperature to avoid unnecessary costs. However, it may be advantageous to apply heated liquids, e.g., from 30 to 100 °C, to facilitate the dissolution of ionic liquids. After the ionic liquid has been removed from the adsorbent, adsorbed aromatics and compounds from the liquid that is being used to remove the ionic liquid can be removed by lowering the pressure to vaporise the adsorbed compounds. Alternatively, the adsorbent may be heated. Evidently, combinations of pressure reduction and heating is feasible. The pressure reduction is suitably such that the reduced pressure ranges from 0.05 to 10 bar. Suitably the pressure is reduced by 10 to 20 bar. Heating may be done such that the temperature of the heated adsorbent is from 50 to 200 °C. If desired, traces of aromatic compounds may be removed subsequently by stripping the adsorbent with a stripping gas, such as carbon dioxide or nitrogen.

In lieu of pressure reduction and/or heating, the adsorbent may alternatively be subjected to stripping with a stripping gas, such as carbon dioxide and/or nitrogen or a C₁₋₄ hydrocarbon, in order to remove any adsorbed aromatic compounds.

Since the process is preferably conducted in a continuous fashion, the process employs more than one adsorption zone so that when fat adsorbent in one adsorption zone is freed from ionic liquid and aromatic compounds, another adsorption zone is used for the contact of the adsorbent with the aromatics-depleted aliphatic stream. Suitably the number of adsorption zones ranges from 2 to 4. The freed ionic liquid, optionally together with aromatic compounds, is preferably recycled to the extraction zone. Thereto the ionic liquid and aromatic compounds may be fed directly into the extraction zone, or be combined with the ionic liquid or mixture before each of those is introduced into the extraction zone.

The skilled person may select the adsorbent from a variety of compounds. The preferred adsorbent is selected from the group consisting of carbon, activated carbon, zeolites, such as zeolite X, Y, clays, such as kaolinite, refractory oxides, such as alumina, silica alumina, silica and titania, porous polymers, such as polystyrene and cellulose acetate, and membranes, e.g. polysiloxanes. The preferred adsorbent is activated carbon. Suitable conditions at which the contact of the aromatics-depleted aliphatic stream with the adsorbent takes place include a temperature of from 20 to 80 °C and a pressure of from 0.1 to 20 bar. It is evident that such a treatment is more energy-friendly than fractionation. This adsorption treatment also prevents thermal decomposition of the ionic liquid.

At least a portion of the aromatics-rich ionic liquid is fed to the stripping zone. Preferably the entire product of the extraction is fed to the stripping zone. In this way the yield of aromatics is optimised and all of the ionic liquid is used for the extraction.

In the stripping zone the aromatics-rich ionic liquid is subjected to a treatment with a non-aqueous stripping gas. By non-aqueous is understood that the stripping gas contains at most 5 vol.% of water or steam. Since some ionic liquids might decompose in contact with water the use of a non-aqueous stripping gas overcomes such problems. The stripping gas suitably contains less than 2 vol. % of water or steam, and is preferably substantially water-free. The stripping gas may be selected from a variety of gases that are inert for ionic liquids. Suitable examples include carbon dioxide and nitrogen. Preferably a hydrocarbon stripping gas is being used.

From the stripping zone a purified stream of ionic liquid is obtained. In accordance with the present process this stream is recycled to the extraction zone. The other product of the stripping zone is an aromatics-rich hydrocarbon stream. This stream comprises the stripping gas and the aromatic compounds that were entrained with the stripping gas. It may be that the aromatics-rich ionic liquid stream from the extraction zone also contains traces of aliphatic hydrocarbons. Such aliphatic hydrocarbons are also entrained with the stripping gas. In order to separate the stripping gas from the entrained aromatic compounds and any aliphatic hydrocarbons that have also been entrained the aromatics-rich hydrocarbon stream is preferably separated into two fractions. The one fraction suitably comprises the stripping gas and any light hydrocarbon that may have been present in the aromatics-rich ionic liquid stream, whereas the aromatic compounds are contained in the other fraction. The separation into a volatile fraction and a liquid fraction may be accomplished in any feasible way. Separation can be accomplished by separation via a membrane which allows the passage of stripping gas molecules but retains aromatic compounds. Preferably the separation is achieved by separating the aromatics-rich hydrocarbon stream into a volatile fraction and a liquid fraction. The volatile fraction contains the stripping gas and the liquid fraction contains the aromatic compounds. Such a separation can be done via fractionation, but is preferably achieved via cooling and condensation of the aromatic compounds. The volatile fraction thus obtained is preferably fed to the stripping zone and used as stripping gas. According to a preferred embodiment of the present invention the stripping gas is a hydrocarbon stripping gas. As such a stripping gas any aliphatic hydrocarbon with a lower boiling point than the aromatic compounds can suitably be applied. Such gas may be a byproduct of the process, e.g. C₁₋₄ cracked products, a designated light gas, such as propane and/or butane, or any other hydrocarbon gas that is easy to separate from the aromatic compounds.

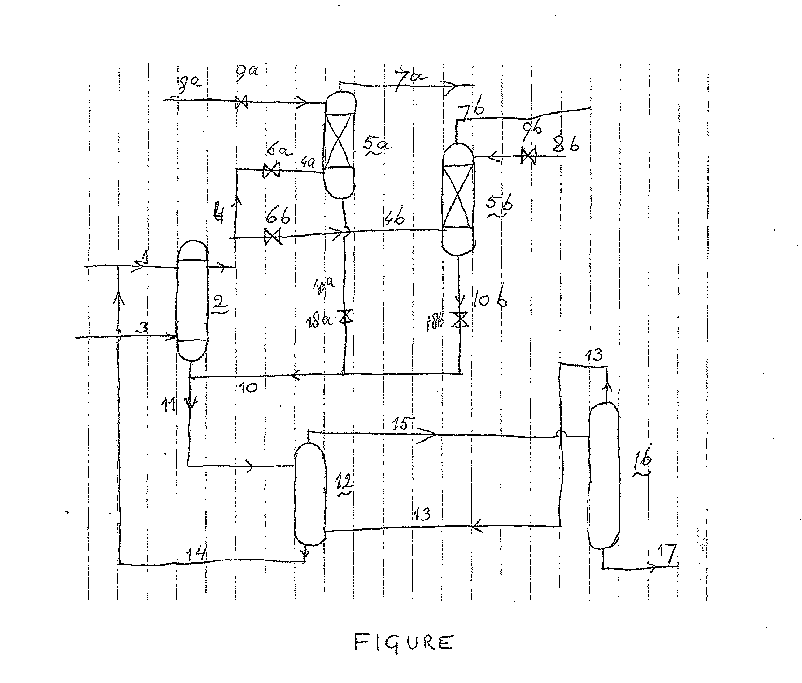

The invention will be further illustrated by means of the Figure that shows a simplified flow scheme of the process according to the present invention.

The Figure shows a hydrocarbon mixture comprising aromatic compounds and at least on aliphatic hydrocarbon being fed into an extraction zone 2 via a line 3. In the extraction zone 2 the hydrocarbon mixture is contacted in counter-current fashion with an ionic liquid that is introduced into the extraction zone 2 via a line 1. The extraction zone may be provided with different internals to promote the transfer process, such as plate trays, bubble caps and various types of baffles. The aliphatic hydrocarbons that result from the extraction are withdrawn via a line 4 that is split into a line 4a and a line 4b. Via valves 6a and 6b the supply of aliphatic hydrocarbons to adsorber columns 5a and 5b can be controlled. When the valve 6b is closed and valve 6a is open, the aliphatic hydrocarbons in lines 4 and 4a are subjected to adsorption in the column 5a. Traces of aromatic compounds and ionic liquid that may be entrained with the aliphatic hydrocarbons are adsorbed on the adsorbent bed that is contained in the adsorber column 5a. In parallel adsorbed aromatic compounds and ionic liquid that has previously been adsorbed by the adsorbent in the adsorber column 5b are being freed from the adsorbent bed in adsorber column 5b by passing a heated aromatic liquid product stream through the bed that has been introduced via a line 8b and an open valve 9b. A similar line 8a and a similar valve 9a for supplying such aromatic liquid stream to the adsorber column 5a are closed. The heated aromatic liquid stream releases the ionic liquid from the adsorbent, and the mixture of aromatic liquid product and ionic liquid is withdrawn via a line 10b.

Remaining aromatic compounds in the adsorber column 5b may be suitably removed by lowering the pressure to vaporise these aromatic compounds. Suitable pressures range from 0.7 to 0.1 bar. Alternatively, remaining aromatic compounds may be removed from the adsorbent bed by using a stripping gas, e.g. nitrogen. The aromatic compounds may subsequently be recovered from the stripping gas by condensation and phase separation.

### (Equipment for the supply and treatment of stripping gas has not been shown in the Figure).

Freed aromatic compounds are discharged from the adsorber column 5b via the line 10b and an open valve 18b and passed to line 10. A similar discharge line 10a and valve 18a from adsorber column 5a are closed. A purified aliphatic hydrocarbons stream is obtained from adsorber column 5a and recovered via a line 7a. A similar line 7b is provided at adsorber column 5b, but is closed when line 7a is used for the recovery of purified aliphatic hydrocarbons.

When the adsorbent bed in the column 5a is rich in aromatic compounds and/or ionic liquid the operations for the columns 5a and 5b switches.

The aromatic compounds and ionic liquid from line 10 are added to a line 11 that withdraws the aromatics-rich ionic liquid from the extraction zone 2. The combined streams are fed to a stripping column 2 where they are contacted with a stripping hydrocarbon gas, e.g., butane, that is passed into the stripping column 12 via a line 13. Purified ionic liquid is withdrawn from the bottom of the stripping column 12 via a line 14 and combined with the ionic liquid in line 1 that is passed to the extraction zone 2.

The aromatics-rich hydrocarbon stream, comprising stripping gas and aromatic compounds and, optionally, traces of aliphatic hydrocarbons, is passed via a line 15 from the stripping column 12 to a condenser 16 wherein the aromatics-rich hydrocarbon stream is separated into a volatile fraction and a liquid fraction. The liquid fraction, i.e., the condensate, contains the aromatic hydrocarbons and this is recovered as product via a line 17. The volatile fraction comprises the stripping gas and, optionally, other gaseous hydrocarbons, and is withdrawn from the condenser 16 via the line 13 and passed to the stripping column for use as stripping gas.

It will be evident that the flow scheme is schematic only and auxiliary equipment, such as compressors, pumps, expanders, control equipment and heating means, like furnaces and heat exchangers, has not been shown.

## Claims

1. Process for the separation of aromatic compounds from a mixture comprising such aromatic compounds and aliphatic hydrocarbons, which process comprises;
(a) contacting the mixture with an ionic liquid in an extraction zone to yield an aromatics-rich ionic liquid stream and an aromatics-depleted aliphatic stream;
(b) feeding at least a portion of the aromatics-rich ionic liquid stream to a stripping zone;
(c) stripping the aromatics-rich ionic liquid with a non-aqueous stripping gas to obtain an aromatics-rich hydrocarbon stream and a purified ionic liquid stream; and
(d) recycling the purified ionic liquid stream to the extraction zone.

2. Process as claimed in claim 1, wherein the mixture comprises from 10 to 75%wt of aromatic compounds, based on the total weight of the mixture.

3. Process as claimed in claim 1 or 2, wherein the aromatic compounds are selected from the group consisting of benzene, toluene, xylene and naphthalene.

4. Process as claimed in any one of claims 1 to 3, wherein the ionic liquid comprises a nitrogen-containing cationic moiety.

5. Process as claimed in claim 4, wherein the nitrogen-containing anionic moiety has been selected from the group consisting of (N-R₁)-pyridinium and 1-R₂-3-R₃-imidazolium, wherein R₁, R₂ and R₃ are alkyl groups with 1 to 6 carbon atoms.

6. Process as claimed in any one of claims 1 to 5, wherein the ionic liquid comprises an anionic moiety comprising an element selected from Groups IB, IIA, IIB, IIIA and VIII of the Periodic Table of Elements.

7. Process as claimed in claim 6, wherein the anionic moiety comprises an element selected from the group consisting of boron, aluminium, gallium, indium, copper, zinc, cobalt, nickel and iron.

8. Process as claimed in any one of claims 1 to 7, wherein the extraction zone is operated at a temperature of 30 to 150°C, a pressure of 0.1 to 20 bar and a residence time of 30 s to 1 hr.

9. Process as claimed in any one of claims 1 to 8, wherein the aromatics-depleted aliphatic stream is contacted with an adsorbent to obtain a purified aliphatic stream and a fat adsorbent comprising ionic liquid.

10. Process as claimed in claim 9, wherein the fat adsorbent is freed from ionic liquid by contacting the fat adsorbent with a liquid stream capable of dissolving the ionic liquid, preferably an aromatic hydrocarbon.

11. Process as claimed in claim 10, wherein the freed ionic liquid, optionally with aromatic compounds, is recycled to the extraction zone.

12. Process as claimed in any one of claims 9 to 11, wherein the adsorbent is activated carbon.

13. Process as claimed in any one of claims 9 to 12, wherein the conditions of the contact of the aromatics-depleted aliphatic stream with the adsorbent include a temperature of from 30 to 80°C and a pressure of from 0.1 to 20 bar.

14. Process as claimed in any one of claims 1 to 13, wherein the aromatics-rich hydrocarbon stream is separated into a volatile fraction and a liquid fraction.

15. Process as claimed in claim 14, wherein the aromatics-rich hydrocarbon stream is separated into a volatile fraction and a liquid fraction by means of condensation.
